# FASCICULE DE BREVET EUROPEEN

(11) **EP 1 555 943 B1**
(45) Date de publication et mention de la délivrance du brevet: **24.02.2010**
(21) Numéro de dépôt: 03807887.9
(22) Date de dépôt: 08.10.2003
(51) Int. Cl.: A61B 17/80, A61B 17/86

(54) **DISPOSITIF D'OSTEOSYNTHESE AUTOBLOQUANT**
SELBSTARRETIERENDE OSTEOSYNTHESEVORRICHTUNG
SELF-LOCKING OSTEOSYNTHESIS DEVICE

(30) Priorité: 09.10.2002 FR 0212534
(43) Date de publication de la demande: 27.07.2005
(73) Titulaire: Biotech International (SARL), 13300 Salon de Provence (FR)
(72) Inventeur: Impellizzeri, Frédéric, 13300 Salon de Provence (FR)
(74) Mandataire: Marek, Pierre
(86) Numéro de dépôt international: PCT/FR2003/002968
(87) Numéro de publication internationale: WO 2004/032751

(56) Documents cités:
- DE-A- 19 629 011
- US-A- 5 275 601
- US-A- 5 578 034
- US-A- 6 129 730

## Description

La présente invention concerne un dispositif d'ostéosynthèse ou d'ostéotomie autobloquant du genre constitué par une plaque de forme appropriée destinée à être fixée sur des fragments osseux, au moyen de vis, pour assurer leur coaptation.

La coaptation des fragments d'os au moyen de plaques de titane ou autre matériau et de vis, pour réaliser une ostéosynthèse est une intervention courante en chirurgie des os, par exemple en chirurgie orthopédique.

Pour l'obtention d'un bon résultat, un serrage permanent des plaques ou implants sur les fragments d'os assemblés par lesdits implants, est nécessaire. Il est donc indispensable que les vis ne puissent se dévisser et reculer, pour empêcher tout déplacement des implants par rapport aux fragments d'os.

D'autre part, il serait souvent souhaitable de pouvoir choisir l'orientation des vis par rapport aux plaques, aussi bien en fonction du positionnement et de la forme des fragments d'os à assembler, que pour améliorer la qualité de l'assemblage.

Pour s'opposer au dévissage et au recul des vis, on a proposé (EP-0.345.133, FR-2.794.963) de loger des organes de blocage à l'entrée des trous de passage des vis prévus dans les plaques, afin de supprimer toute possibilité de mouvement axial de recul desdites vis, après leur vissage dans la matière osseuse. Par exemple, il est prévu, dans le document EP-0.345.133, d'utiliser des contre-vis filetées extérieurement et coopérant avec un filetage complémentaire ménagé à l'entrée des trous de passage des vis dont sont munies les plaques, de sorte que la tête desdites vis se trouve calée contre une contre-vis et que ces dernières ne peuvent se déplacer axialement par rapport auxdites plaques, ce blocage assurant ainsi la permanence de l'appui de la plaque sur les fragments d'os.

Ces dispositifs proposés par quelques fabricants, représentent, à ce jour, les solutions les plus sûres en terme de blocage. Toutefois, ces dispositifs relativement complexes nécessitent l'utilisation de plaques présentant une épaisseur relativement importante totalement incompatible avec une utilisation pour des interventions sur des os de la main ou du pied, pour lesquelles l'épaisseur des plaques doit être la plus réduite possible, compte tenu de la petite taille des os concernés.

Dans le document EP-0.345.133, est encore montré un dispositif de solidarisation de deux éléments tels qu'un implant et un os, suivant lequel l'implant comporte des trous de passage des vis dont les axes sont orientés obliquement les uns par rapport aux autres, de sorte que les vis traversant ces trous ont des orientations rigoureusement imposées par la direction desdits axes. Un tel dispositif ne peut être envisagé que pour la réduction de fractures identiques, car autrement il faudrait prévoir autant de modèles de plaques que de cas possibles de fractures, ce qui est pratiquement impossible ; il n'offre en effet aucune possibilité de choisir l'orientation des vis en fonction des problèmes rencontrés en chirurgie orthopédique.

Dans le document WO-00/66012, est décrite une plaque pour ostéosynthèse pouvant être bloquée, suivant laquelle les vis et les trous de passage des vis prévus dans la plaque, sont munis, respectivement, d'un filetage de blocage et d'un profil d'engrènement sensés permettre l'introduction des vis dans la plaque, de manière inclinée. La réalisation pratique d'un tel dispositif parait difficile et son efficacité n'a pas été établie, semble-t-il.

Dans le document DE 196 29 011 A1 est montré un dispositif d'ostéosynthèse autobloquant du genre comprenant une plaque munie de trous pour le passage de vis de fixation, la plaque d'ostéosynthèse étant constituée par une plaque composite dont les pourtours des trous sont constitués par des inserts, la plaque d'ostéosynthèse étant exécutée, au moins dans les zones délimitant les trous de passage des vis, dans un matériau présentant des propriétés mécaniques autorisant un auto-taraudage du pourtour desdits trous au moyen de vis taraudeuses utilisables pour la fixation de ladite plaque.

D'une façon générale, dans le domaine de l'ostéosynthèse de petits os nécessitant l'utilisation de plaques de petites dimensions, les dispositifs actuellement présents sur le marché, ne rendent pas possible un débattement angulaire entre les vis et la plaque, de sorte que celles-ci se trouvent ainsi obligatoirement positionnées perpendiculairement à la plaque. Or, dans certains cas, il serait souhaitable de pouvoir incliner une ou plusieurs vis, pour utiliser un ou des os de meilleure qualité pour le vissage.

L'invention a notamment pour but de remédier aux insuffisances susmentionnées des systèmes d'ostéosynthèse utilisant des plaques et des vis, notamment en raison du fait que les dispositifs existant pour de l'orthopédie lourde (traitement des gros traumatismes), ne sont pas transposables à la chirurgie des mains et des pieds pour laquelle la dimension des plaques utilisables se réduit considérablement.

Selon l'invention, ce but est atteint grâce à un dispositif d'ostéosynthèse comprenant une plaque munie de trous pour le passage de vis et des vis pour la fixation de cette plaque sur un support osseux, ce dispositif étant remarquable en ce que la plaque d'ostéosynthèse est exécutée, au moins dans les zones délimitant les trous de passage des vis, dans un matériau présentant des propriétés mécaniques autorisant un auto-taraudage du pourtour des trous au moyen de vis taraudeuses utilisables pour la fixation de ladite plaque.

La plaque d'ostéosynthèse est constituée par une plaque composite dont les pourtours des trous sont constitués par des inserts exécutés dans une matière plastique biocompatible, et insérés dans des trous que présente la partie restante de la plaque réalisée en métal.

Selon une autre disposition caractéristique, les pourtours des trous sont constitués par des inserts exécutés en Polyétheréthercétone (PEEK) et la partie restante de la plaque est réalisée en titane.

Selon une autre disposition caractéristique, la tête des vis présente un filetage taraudeur conique.

Grâce aux dispositions caractéristiques ci-dessus, la tête auto-taraudeuse des vis creuse son propre sillon hélicoïdal récepteur dans le pourtour des trous dans lesquels elles sont engagées, de sorte que lesdites vis se trouvent ensuite automatiquement bloquées dans la plaque lorsque leur tête se trouve serrée dans son logement.

D'autre part, la plaque d'ostéosynthèse selon l'invention autorise une angulation sélective des vis par rapport à l'axe des trous de ladite plaque, en fonction des besoins.

Outre l'obtention des résultats très intéressants susmentionnés, le dispositif d'ostéosynthèse selon l'invention procure plusieurs avantages :
- il est de conception simple, de sorte qu'il peut être fabriqué de manière économique ;
- sa mise en place est aisée ;
- il est très fiable à l'usage ;
- il peut être utilisé pour assurer le blocage des vis de fixation des plaques d'ostéosynthèse de petites tailles, ce que ne permettent pas les systèmes actuellement proposés sur le marché.

Ce dispositif d'ostéosynthèse répond donc parfaitement aux attentes des chirurgiens en termes de facilité de mise en place et de fiabilité à l'usage.

Les buts, caractéristiques et avantages ci-dessus, et d'autres encore, ressortiront mieux de la description qui suit et des dessins annexés dans lesquels:
La figure 1 est une vue en perspective, avec arrachement partiel, d'une plaque d'ostéosynthèse selon l'invention, dont la conformation est donnée à titre d'exemple seulement.

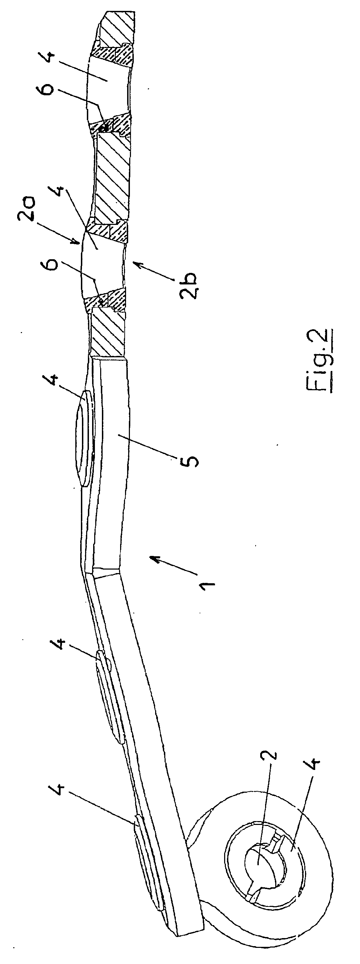

La figure 2 est une vue de côté, avec coupe partielle, de la figure 1.

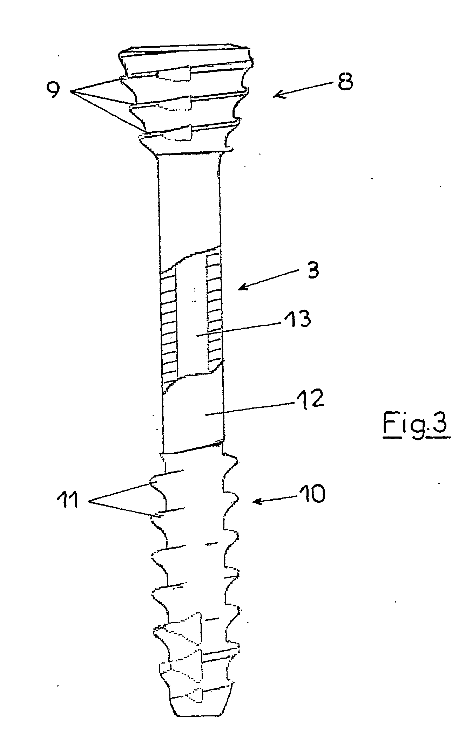

La figure 3 est une vue en élévation, avec coupe axiale partielle et à échelle agrandie, d'une vis à tête conique utilisable pour la mise en oeuvre de l'invention.

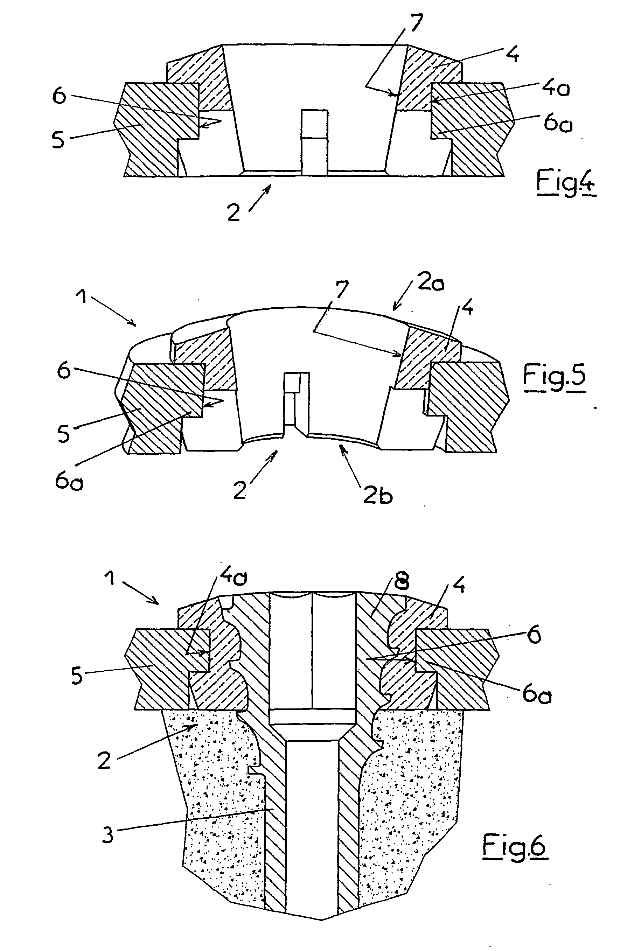

La figure 4 est une vue de détail montrant, en coupe axiale, un insert implanté dans une plaque d'ostéosynthèse pour la réception d'une vis taraudeuse.
La figure 5 est une vue en coupe et en perspective de cet insert.
La figure 6 est une vue illustrant une vis taraudeuse vissée dans cet insert et dans un fragment d'os.
La figure 7 est une vue en élévation, avec coupes partielles, illustrant la fixation d'une plaque d'ostéosynthèse selon l'invention pour la coaptation de fragments d'os, deux des vis de fixation (à droite et à gauche) sont montrées complètement vissées dans les fragments d'os et dans la plaque, une troisième vis (au centre) étant illustrée en cours de mise en place.

On se reporte auxdits dessins pour décrire un exemple de réalisation intéressant, bien que nullement limitatif, du dispositif d'ostéosynthèse autobloquant selon l'invention.

Ce dispositif comprend une plaque 1 munie de trous traversants 2 et des vis de fixation 3.

Selon l'invention, la plaque 1 est exécutée, au moins dans les zones délimitant les trous 2 de passage des vis 3, dans un matériau 4 présentant des propriétés mécaniques permettant un auto-taraudage du pourtour desdits trous au moyen de vis taraudeuses utilisables pour la fixation de ladite plaque sur la matière osseuse.

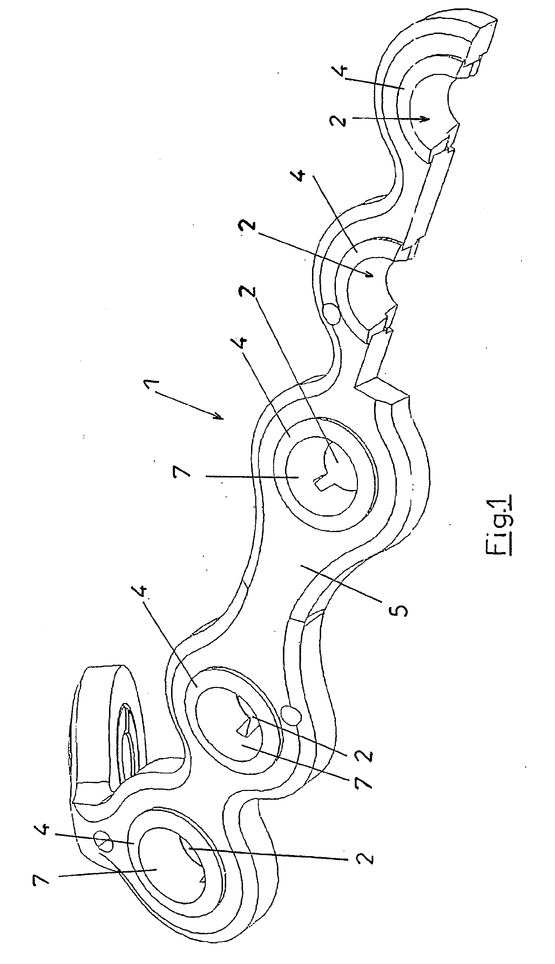

La plaque 1 peut présenter toute conformation adaptée aux cas de réductions de fractures ou de chirurgie restauratrice à traiter ; la forme illustrée à la figure 1 n'est donc qu'un exemple de conformation possible. Il en va de même pour l'emplacement des trous 2 dans la plaque.

La plaque 1 est constituée par une plaque composite dont les pourtours 4 des trous 2 sont exécutés dans une matière plastique biocompatible, la partie ou surface restante 5 de ladite plaque étant réalisée en métal.

Les pourtours 4 des trous 2 peuvent être exécutés en un polymère thermoplastique à hautes performances. De manière préférée et avantageuse, les pourtours 4 des trous 2 sont exécutés en Polyétheréthercétone (PEEK) qui possède des propriétés mécaniques très élevées et qui peut être usiné, comme un métal.

La partie ou surface restante 5 des plaques 1 peut être exécutée en acier inoxydable ; de manière préférée et avantageuse, elle est réalisée en titane.

La fixation solide des inserts constitués par les pourtours 4 des trous 2 dans les trous 6 ménagés dans la plaque de base métallique 5 peut s'effectuer par une technique de surmoulage ayant pour avantage d'assurer un contact intime entre les deux matériaux.

Toutefois, de manière préférée, la mise en place des inserts en PEEK 4 dans les trous 6 de la plaque métallique 5 est opérée au moyen d'un assemblage mécanique. Les inserts en PEEK 4 sont engagés, par déformation et pression dans les trous de la plaque métallique et se trouvent ensuite maintenus dans ces derniers. Par exemple, les inserts 4 peuvent comporter une gorge périphérique 4a dans laquelle vient s'engager un rebord supérieur 6a des trous 6 de la plaque 1, lorsque les inserts sont enfoncés dans lesdits trous. Lors du vissage des vis 3, les inserts 4 se déforment et sont comprimés contre les bords des trous 6 de la plaque métallique 5, ce qui contribue à la solidité de l'ancrage desdits inserts dans ladite plaque métallique.

Un moyen est prévu pour empêcher toute possibilité de rotation des inserts 4 dans les trous 6 de la plaque 5, lors du vissage des vis dans lesdits inserts. Ce moyen peut, par exemple, être constitué par une ou plusieurs stries ménagée(s) dans la surface interne cylindrique des trous 6 de la plaque 5, parallèlement à l'axe de ces derniers.

Grâce aux moyens précédemment décrits, les inserts 4 se trouvent solidement liés à la plaque 1, sans possibilité de mouvement axial ou de rotation.

Les trous 2 ont une forme conique. La grande ouverture 2a des trous 2 accessible du côté externe de la plaque 1 constitue l'entrée de ceux-ci, tandis que la petite ouverture 2b desdits trous 2 débouche sur le côté interne de la plaque destiné à être appliquée sur les parties d'os à assembler. La paroi conique 7 des trous 2 des inserts 4 est lisse et ne comporte aucun filetage avant utilisation de la plaque d'ostéosynthèse.

Les vis 3 utilisables pour la fixation de la plaque d'ostéosynthèse 1 qui vient d'être décrite, comportent une partie proximale ou tête 8 dotée d'un filetage taraudeur conique 9 dont le diamètre croît en direction de l'extrémité proximale desdites vis.

Ces vis sont, par exemple, du genre décrit dans le document WO 98/40024. Elles comprennent une partie distale 10 pourvue d'un filetage cylindrique 11, une partie proximale ou tête 8 dotée d'un filetage taraudeur conique 9 et, de préférence, un tronçon lisse intermédiaire 12 disposé entre lesdites partie distale 10 et proximale 8.

Les vis 3 sont exécutées en tout matériau biocompatible convenable et elles peuvent être avantageusement de type "canulée", c'est-à-dire munie d'un canal axial 13 s'étendant d'une extrémité à l'autre desdites vis, pour le passage d'une broche.

Le diamètre nominal du filetage cylindrique 11 de la partie distale 10 des vis est inférieur au diamètre de la petite ouverture 2b des trous 2, de sorte que ladite partie distale des vis peut traverser librement lesdits trous lors de la fixation de la plaque d'ostéosynthèse 1 sur des os.

La plaque composite d'ostéosynthèse selon l'invention permet un vissage des vis 3 avec une certaine angulation par rapport aux axes des trous 2 de ladite plaque.

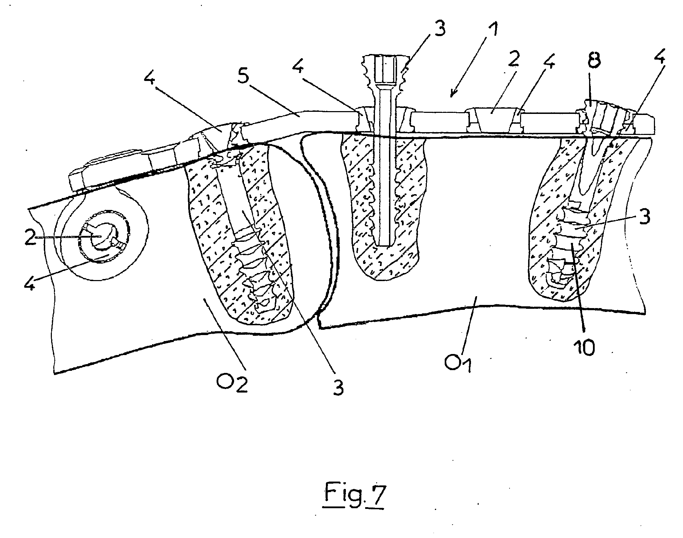

La figure 7 illustre un exemple de coaptation de morceaux d'os O1 et O2 au moyen du dispositif d'ostéosynthèse selon l'invention.

Sur les parties droite et gauche de la figure, on voit la plaque d'ostéosynthèse 1 pressée contre des morceaux d'os 01 et 02, au moyen de deux vis 3 complètement vissées, dont la partie distale 10 est vissée dans la matière osseuse 14 et dont la partie proximale 8 est vissée dans des inserts 4 de ladite plaque, les vis ayant des orientations différentes et se trouvant bloquées grâce aux dispositions précédemment décrites.

Sur la partie centrale de la figure, on voit une vis 3 en cours de vissage.

Comme indiqué précédemment, l'invention permet d'obtenir un blocage des vis supprimant toute possibilité de recul après leur vissage dans la matière osseuse. Ce blocage est d'autant plus efficace que la tête conique à filetage taraudeur des vis produit un effet de coincement résultant de l'assemblage conique des vis et des inserts.

## Revendications

1. Dispositif d'ostéosynthèse autobloquant du genre comprenant une plaque (1) munie de trous (2) pour le passage de vis de fixation (3), ladite plaque d'ostéosynthèse (1) étant constituée par une plaque composite dont les pourtours (4) des trous (2) sont constitués par des inserts exécutés dans une matière plastique biocompatible présentant des propriétés mécaniques autorisant un auto-taraudage du pourtour (4) desdits trous (2) au moyen de vis taraudeuses utilisables pour la fixation de la plaque, lesdits inserts étant insérés dans des trous (6) que présente la partie restante de ladite plaque réalisée en métal.

2. Dispositif suivant la revendication 1, **caractérise en ce que** les pourtours (4) des trous (2) sont constitués par des inserts exécutés en polymère thermoplastique à haute performance.

3. Dispositif selon la revendication 2, **caractérise en ce que** les pourtours (4) des trous (2) sont constitués par des inserts exécutés en polyétheréthercétone (PEEK).

4. Dispositif selon l'une quelconque des revendications 1 à 3, **caractérisé en ce que** les trous (2) de passage des vis de fixation (3) ont une forme conique.

5. Dispositif selon l'une quelconque des revendications 1 à 4 **caractérisé en ce que** la partie restante de la plaque ou plaque de base (5) est exécutée en titane.

6. Dispositif suivant l'une quelconque des revendications 1 à 5, **caractérisé en ce que** les inserts (4) constituant le pourtour des trous (2) sont intégrés à la partie restante de la plaque ou plaque de base (5), par surmoulage.

7. Dispositif selon l'une quelconque des revendications à 5, **caractérisé en ce que** les inserts (4) constituant le pourtour des trous (2) sont intégrés à la partie restante de la plaque ou plaque de base (5) au moyen d'un assemblage mécanique.

8. Dispositif suivant l'une quelconque des revendications 1 à 7, **caractérisé en ce qu'**il comprend également des vis taraudeuses (3) comportant une partie proximale ou tête (8) dotée d'un filetage taraudeur conique (9) dont le diamètre croît en direction de l'extrémité proximale desdites vis.

9. Dispositif selon l'une quelconque des revendications 1 à 8, **caractérisé en ce qu'**il comporte un moyen s'opposant à la rotation des inserts (4) engagés dans les trous (6) de la plaque (5).

## Claims

1. A self-locking osteosynthesis device of the kind comprising a plate (1) provided with holes (2) for passing fixing screws (3), said osteosynthesis plate (1) being formed by a composite plate in which the peripheries (4) of the holes (2) are formed by inserts made of a biocompatible plastic material having mechanical properties permitting self-tapping of the periphery (4) of the holes (2) by means of tapping screws which can be used for fixing the plate, said inserts being inserted into holes (6) in the remaining part of said plate that is made of metal.

2. A device according to claim 1 **characterised in that** the peripheries (4) of the holes (2) are formed by inserts made of high-performance thermoplastic polymer.

3. A device according to claim 1 **characterised in that** the peripheries (4) of the holes (2) are formed by inserts made of polyether ether ketone (PEEK).

4. A device according to any one of claims 1 to 3 **characterised in that** the holes for passing the fixing screws (3) are conical in shape.

5. A device according to any one of claims 1 to 4 **characterised in that** the remaining part of the plate or base plate (5) is made of titanium.

6. A device according to any one of claims 1 to 5 **characterised in that** the inserts (4) forming the periphery of the holes (2) are integrated in the remaining part of the plate or base plate (5) by being moulded thereon.

7. A device according to any one of claims 1 to 5 **characterised in that** the inserts (4) forming the periphery of the holes (2) are integrated in the remaining part of the plate or base plate (5) by means of mechanical assembly.

8. A device according to any one of claims 1 to 7 **characterised in that** it also comprises tapping screws (3) comprising a head or proximal part (8) provided with a conical thread tapping means (9), the diameter of which increases in the direction of the proximal end of said screws.

9. A device according to any one of claims 1 to 8 **characterised in that** it comprises a means for resisting rotation of the inserts (4) which are engaged in the holes (6) in the plate (5).

## Patentansprüche

1. Selbstarretierende Osteosynthesevorrichtung der Art, die eine Platte (1) umfasst, die mit Löchern (2) für das Hindurchführen von Befestigungsschrauben (3) versehen ist, wobei die Osteosyntheseplatte (1) von einer Verbundplatte gebildet wird, bei welcher die Umfänge (4) der Löcher (2) von Einsätzen gebildet werden, die aus einem biokompatiblen Kunststoff ausgeführt sind, der mechanische Eigenschaften aufweist, die ein selbsttätiges Gewindeschneiden der Umfänge (4) der Löcher (2) mittels Gewindeschneidschrauben gestatten, die für die Befestigung der Platte verwendbar sind, wobei die Einsätze in Löcher (6) eingesetzt sind, die der übrige, aus Metall gebildete Teil der Platte aufweist.

2. Vorrichtung nach Anspruch 1, **dadurch gekennzeichnet, dass** die Umfänge (4) der Löcher (2) aus Einsätzen gebildet sind, die aus einem thermoplastischen Hochleistungspolymer ausgeführt sind.

3. Vorrichtung nach Anspruch 2, **dadurch gekennzeichnet, dass** die Umfänge (4) der Löcher (2) aus Einsätzen gebildet sind, die aus Polyetheretherketon (PEEK) ausgeführt sind.

4. Vorrichtung nach einem der Ansprüche 1 bis 3, **dadurch gekennzeichnet, dass** die Löcher (2) zum Hindurchführen der Befestigungsschrauben (3) eine konische Form aufweisen.

5. Vorrichtung nach einem der Ansprüche 1 bis 4, **dadurch gekennzeichnet, dass** der übrige Teil der Platte bzw. Grundplatte (5) aus Titan ausgeführt ist.

6. Vorrichtung nach einem der Ansprüche 1 bis 5, **dadurch gekennzeichnet, dass** die Einsätze (4), die den Umfang der Löcher (2) bilden, in den übrigen Teil der Platte bzw. Grundplatte (5) mittels Überformung integriert sind.

7. Vorrichtung nach einem der Ansprüche 1 bis 5, **dadurch gekennzeichnet, dass** die Einsätze (4), die den Umfang der Löcher (2) bilden, in den übrigen Teil der Platte bzw. Grundplatte (5) mittels mechanischer Montage integriert sind.

8. Vorrichtung nach einem der Ansprüche 1 bis 7, **dadurch gekennzeichnet, dass** sie des Weiteren Gewindeschneidschrauben (3) umfasst, die einen proximalen Teil bzw. Kopf (8) umfassen, der mit einem konischen Schneidgewinde (9) versehen ist, dessen Durchmesser in Richtung auf das proximale Ende der Schrauben zunimmt.

9. Vorrichtung nach einem der Ansprüche 1 bis 8, **dadurch gekennzeichnet, dass** sie ein Mittel umfasst, das der Drehung der Einsätze (4) entgegenwirkt, die in die Löcher (6) der Platte (5) eingesetzt sind.
